# EUROPÄISCHE PATENTANMELDUNG

(11) **EP 4 465 071 A1**
(43) Veröffentlichungstag der Anmeldung: **20.11.2024**
(21) Anmeldenummer: 23173931.9
(22) Anmeldetag: 17.05.2023
(51) Int. Cl.: G01R 33/56, G06T 7/00

(54) **VERFAHREN ZUM BEREITSTELLEN VON ZUMINDEST EINER BEWERTUNGSKENNGRÖSSE EINER BILDQUALITÄT VON ZUMINDEST EINEM MAGNETRESONANZBILD**

(71) Anmelder: Siemens Healthineers AG, 91301 Forchheim (DE); Friedrich-Alexander-Universität Erlangen-Nürnberg, 91054 Erlangen (DE)
(72) Erfinder: Saksena, Vanya, 91052 Erlangen (DE); Arroyo Camejo, Silvia Bettina, 90763 Fürth (DE); Hossbach, Julian, 91058 Erlangen (DE)
(74) Vertreter: Siemens Healthineers Patent Attorneys

(57) **Zusammenfassung**

Die Erfindung geht aus von einem Verfahren zu einem Bereitstellen von zumindest einer Bewertungskenngröße einer Bildqualität von zumindest einem Magnetresonanzbild, umfassend die folgenden Schritte:
- Bereitstellen von zumindest einem Magnetresonanzbild für eine Bewertung der Bildqualität, wobei das Bereitstellen an eine Qualitätseinheit erfolgt,
- Bestimmen von zumindest zwei unterschiedlichen Qualitätsmaßen des zumindest einen Magnetresonanzbilds, wobei die Qualitätseinheit zwei oder mehr Qualitätsmodule umfasst und jedes der zwei oder mehr Qualitätsmodule zur Bestimmung eines der zwei oder mehr unterschiedlichen Qualitätsmaße ausgebildet ist, und Bereitstellen der zwei oder mehr Qualitätsmaße an eine Bewertungseinheit,
- Ermitteln der zumindest einer Bewertungskenngröße mittels der Bewertungseinheit, wobei die zumindest eine Bewertungskenngröße anhand der zwei oder mehr unterschiedlichen Qualitätsmaße ermittelt wird, und
- Bereitstellen der zumindest einen Bewertungskenngröße.

## Beschreibung

Die vorliegende Erfindung betrifft ein Verfahren zu einem Bereitstellen zumindest einer Bewertungskenngröße einer Bildqualität von zumindest einem Magnetresonanzbild. Des Weiteren betrifft die vorliegende Erfindung ein System mit einer Magnetresonanzvorrichtung, einer Qualitätseinheit und einer Bewertungseinheit, wobei das System zu einem Ausführen eines Verfahrens zu einem Bereitstellen zumindest einer Bewertungskenngröße einer Bildqualität von zumindest einem Magnetresonanzbild ausgebildet ist.

Unabhängig vom grammatikalischen Geschlecht eines bestimmten Begriffes sind Personen mit männlicher, weiblicher oder anderer Geschlechteridentität mit umfasst.

Die Magnetresonanztomographie (MRT) ist eine leistungsstarke Bildgebungsmethode, die einen hohen Weichteilkontrast ohne schädliche Strahlung, insbesondere Röntgenstrahlung, aufweist. Bei MRT-Bildern kann es jedoch zu Problemen mit der Bildqualität kommen, wie z. B. bei Artefakten, die insbesondere auf unzureichende Einstellungen oder mangelnde Patientenkonformität zurückzuführen sind. Bisher wird die Bildqualitätssicherung während der Messzeit von einem medizinischen Bedienpersonal manuell durchgeführt. Ein derartiger Prozess der manuellen Bildqualitätssicherung ist jedoch sehr zeitaufwändig. Zudem unterliegt ein derartiger Prozess der manuellen Bildqualitätssicherung subjektiven Kriterien des medizinischen Bedienpersonals.

Bei der MRT variieren der Bildeindruck und die Eigenschaften stark über verschiedene Kontraste, Anatomien, Patienten, Magnetresonanzvorrichtungen und Standorte. Darüber hinaus haben Radiologen individuelle Präferenzen für eine akzeptable Bildqualität und ihre Empfindlichkeit gegenüber der Schwere des Bildqualitätsproblems. Trotz großer Fortschritte auf dem Gebiet der automatisierten Bildqualitätsbewertung bleibt es eine große Herausforderung, eine Methode zu finden, die robust genug ist, um die Variabilität des MR-Bildeindrucks zu berücksichtigen und gleichzeitig eine benutzerspezifische Toleranzanpassung zu ermöglichen.

Falsche Qualitätsbeurteilungen passieren vor allem in Stresssituationen und bei unerfahrenen medizinischen Bedienpersonal. Eine automatisierte und objektive Methode zur Bewertung der Bildqualität (IQA), die am Scanner verfügbar ist, könnte einen großen Nutzen bieten, um die Qualität der aufgenommenen Bilder sicherzustellen.

Der vorliegenden Erfindung liegt daher insbesondere die Aufgabe zugrunde, eine schnelle und robuste Bewertung einer Bildqualität während einer Magnetresonanzuntersuchung bereitzustellen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einem Bereitstellen von zumindest einer Bewertungskenngröße einer Bildqualität von zumindest einem Magnetresonanzbild, umfassend die folgenden Schritte:
- Bereitstellen von zumindest einem Magnetresonanzbild für eine Bewertung der Bildqualität, wobei das Bereitstellen an eine Qualitätseinheit erfolgt,
- Bestimmen von zumindest zwei unterschiedlichen Qualitätsmaßen des zumindest einen Magnetresonanzbilds, wobei die Qualitätseinheit zwei oder mehr Qualitätsmodule umfasst und jedes der zwei oder mehr Qualitätsmodule zur Bestimmung eines der zwei oder mehr unterschiedlichen Qualitätsmaße ausgebildet ist, und Bereitstellen der zwei oder mehr Qualitätsmaße an eine Bewertungseinheit,
- Ermitteln der zumindest einen Bewertungskenngröße mittels der Bewertungseinheit, wobei die zumindest eine Bewertungskenngröße anhand der zwei oder mehr unterschiedlichen Qualitätsmaße ermittelt wird, und
- Bereitstellen der zumindest einen Bewertungskenngröße.

Das Bereitstellen von zumindest einem Magnetresonanzbild umfasst bevorzugt ein Erfassen des zumindest einen Magnetresonanzbilds während einer Magnetresonanzuntersuchung und ein Bereitstellen des erfassten Magnetresonanzbilds an die Qualitätseinheit. Zudem kann das Bereitstellen auch ein Bereitstellen von zumindest einem gespeicherten Magnetresonanzbild aus einer Speichereinheit umfassen. Das Bereitstellen erfolgt bevorzugt mittels einer Bereitstellungseinheit, die von einer Recheneinheit der Magnetresonanzvorrichtung umfasst ist.

Die Qualitätseinheit umfasst die zwei oder mehr unterschiedlichen Qualitätsmodule. Bevorzugt umfasst die Qualitätseinheit eine Vielzahl von unterschiedlichen Qualitätsmodulen. Jedes der unterschiedlichen Qualitätsmodule ist zu einer Bestimmung eines Qualitätsmaßes, insbesondere eines einzigen Qualitätsmaßes, ausgebildet. Die einzelnen Qualitätsmodule und damit die unterschiedlichen Qualitätsmaße sind dabei unabhängig voneinander und beurteilen dabei jeweils unterschiedliche Bildeigenschaften des zumindest einen Magnetresonanzbilds. Beispielsweise können die unterschiedlichen Bildeigenschaften ein Rauschen und/oder unterschiedliche Rauschparameter des zumindest einen Magnetresonanzbildes umfassen.

Die unterschiedlichen Qualitätsmodule können beispielsweise die folgenden Qualitätseigenschaften bei der Bestimmung des jeweiligen Qualitätsmaßes berücksichtigen:
- Normalisiertes Quadratisches Mittel (normalized root mean square - NRMS), auch Streuindex genannt, umfasst einen statistischer Fehlerindikator,
- Spitzen-Signal-Rausch-Verhältnis (Peak signal-to-noise ratio - PSNR), umfasst das Verhältnis zwischen der maximal möglichen Leistung eines Signals und der Stärke des störenden Rauschens,
- DCT (discrete cosine transform) subbands similarity (DSS). DSS nutzt wichtige Merkmale der menschlichen visuellen Wahrnehmung aus, indem sie Änderungen in Strukturinformationen in Teilbändern im Bereich der DCT misst und die Qualitätsschätzungen für diese Teilbänder gewichtet.
- Gradient magnitude similarity deviation (GMSD). Bildverläufe reagieren empfindlich auf Bildverzerrungen, während verschiedene lokale Strukturen in einem verzerrten Bild unterschiedlich stark beeinträchtigt werden. Mittels der GMSD wird die pixelweise Gradienten-Magnituden-Ähnlichkeit (GMS) in Kombination mit der Standardabweichung verwendet ein Bildqualitätsindex berechnet.
- Haar wavelet-based Perceptual similarity index (HaarPSI) ist ein Ähnlichkeitsmaß für Bilder, das darauf abzielt, die wahrnehmungsmäßige Ähnlichkeit zweier Bilder in Bezug auf einen menschlichen Betrachter korrekt einzuschätzen.
- Mean deviation similarity index (MDSI) verwendet die Gradientengröße zur Messung struktureller Verzerrungen und Farbigkeitsmerkmale zur Messung von Farbverzerrungen in Bildern. Diese beiden Ähnlichkeitskarten werden kombiniert, um eine Gradienten-Chromatizitäts-Ähnlichkeitskarte zu bilden und daraus die endgültige Qualitätsbewertung zu berechnen.
- Mean structural similarity index measure (MSSIM) misst die Ähnlichkeit zwischen zwei gegebenen Bildern.
- Multi-scale structural similarity index measure (MSSSIM) umfasst eine fortgeschrittenere Form der SSIM, die über mehrere Skalen in einem Prozess mit mehrstufiger Verringerung der Abtastung durchgeführt wird.
- Visual information fidelity (VIF) umfasst einen vollständiger Referenzindex zur Bewertung der Bildqualität, der auf natürlichen Szenenstatistiken und der Vorstellung von Bildinformationen basiert, die vom menschlichen visuellen System extrahiert werden.
- Visual saliency-based index (VSI). Hierbei wird die visuelle Ausprägung bei der Berechnung einer lokalen Qualitätskarte des verzerrten Bildes verwendet. Zudem wird die visuelle Ausprägung auch beim Zusammenfassen des Qualitätsfaktors als Gewichtungsfunktion verwendet, um die Bedeutung einer lokalen Region widerzuspiegeln.
- Deep image structure and texture similarity (DISTS) beschreibt eine Bildqualitätsmethode, die Korrelationen von räumlichen Durchschnittswerte ("Texturähnlichkeit") mit Korrelationen in Merkmalskarten ("Strukturähnlichkeit") kombiniert.
- Learned perceptual image patch similarity (LPIPS) wird verwendet, um die Wahrnehmungsähnlichkeit zwischen zwei Bildern zu beurteilen. LPIPS berechnet im Wesentlichen die Ähnlichkeit zwischen den Aktivierungen zweier Bild-Patches für ein vordefiniertes Netzwerk.
- Perceptual image error metric (PieAPP) misst den Wahrnehmungsfehler eines verzerrten Bildes in Bezug auf eine Referenz und den zugehörigen Datensatz.
- Total variation (TV) identifiziert mehrere leicht unterschiedliche Konzepte, die sich auf die Struktur des Wertebereichs einer Funktion oder eines Maßes beziehen.
- Blind referenceless image spatial quality evaluator (BRISQUE) ist ein Modell, das nur die Bildpixel zur Berechnung von Merkmalen verwendet. Es hat sich als äußerst effizient erwiesen, da zur Berechnung seiner Merkmale keine Transformation erforderlich ist. Es basiert auf dem räumlichen NSS-Modell (Natural Scene Statistics) lokal normalisierter Luminanzkoeffizienten im räumlichen Bereich sowie auf dem Modell für paarweise Produkte dieser Koeffizienten.
- Natural image quality evaluator (NIQE) misst den Abstand zwischen den aus einem Bild berechneten NSS-basierten Merkmalen und den Merkmalen, die aus einer Bilddatenbank erhalten wurden, die zum Trainieren des Modells verwendet wurde. Die Merkmale werden als mehrdimensionale Gaußsche Verteilungen modelliert.

Bevorzugt berücksichtigt jedes Qualitätsmodul zur Bestimmung des jeweiligen Qualitätsmaßes eine einzige der Qualitätseigenschaften. Zudem kann die Qualitätseinheit weitere Qualitätsmodule mit weiteren Qualitätseigenschaften umfassen. Die einzelnen Qualitätsmaße können beispielweise eine Zahl umfassen, deren Wert ein Maß für die Qualität darstellt. Beispielsweise kann die Zahl einen Wert zwischen 0 und 1 umfassen, und je größer der Wert der Zahl ist, desto höher ist für diese Kategorie die ermittelte Qualität. Sofern die ermittelte Zahl umso kleiner ist, je größer die Qualität ist, kann auch ein Wert von 1 minus die Zahl ermittelt werden, um in der Bewertungseinheit eine einheitliche Beurteilung zu erreichen.

Die einzelnen Qualitätsmodule bestimmen jedes für sich ein Qualitätsmaß und/oder eine Bildeigenschaft hinsichtlich einer Qualität. Die Erfinder haben erkannt, dass bereits drei bis vier bereitgestellte Qualitätsmaße aus drei bis vier Qualitätsmodulen für eine robuste Beurteilung der Bildqualität ausreichend sind. Zudem ist es aber auch denkbar, dass mehr als vier bereitgestellte Qualitätsmaße von mehr als vier Qualitätsmodulen für die Beurteilung der Bildqualität verwendet werden.

Zur Bestimmung der einzelnen Qualitätsmaße können die einzelnen Qualitätsmodule auch jeweils ein trainiertes maschinelles Lernverfahren umfassen. Im Allgemeinen ahmt ein trainiertes maschinelles Lernverfahren kognitive Funktionen nach, die Menschen mit anderen menschlichen Gedanken assoziieren. Insbesondere durch das Training auf Basis von Trainingsdaten ist das maschinelle Lernverfahren in der Lage, sich an neue Gegebenheiten anzupassen und Muster zu erkennen und zu extrapolieren. Ein anderer Begriff für "trainiertes maschinelles Lernverfahren" ist "trainierte Funktion" oder "trainiertes maschinelles Lernmodell". Generell können Parameter eines maschinelles Lernverfahren mittels Training angepasst werden. Insbesondere können ein überwachtes Training, ein teilüberwachtes Training, ein unüberwachtes Training, Reinforcement Learning und/oder aktives Lernen eingesetzt werden. Darüber hinaus kann Repräsentationslernen (ein alternativer Begriff ist "Feature Learning") verwendet werden. Insbesondere die Parameter der maschinelles Lernverfahren können durch mehrere Trainingsschritte iterativ angepasst werden. Des Weiteren innerhalb des Trainings eines neuronalen Netzes kann der Backpropagation-Algorithmus verwendet werden. Insbesondere kann ein maschinelles Lernverfahren ein neuronales Netz, eine Unterstützungsvektormaschine, einen Entscheidungsbaum und/oder ein Bayes'sches Netzwerk umfassen, und/oder das maschinelles Lernverfahren kann auf k-Means-Clustering, Q-Learning, genetischen Algorithmen und/oder Assoziationsregeln basieren. Insbesondere kann ein neuronales Netz ein tiefes neuronales Netz, ein faltungsneuronales Netz oder ein faltungstiefes neuronales Netz sein. Darüber hinaus kann ein neuronales Netzwerk ein kontradiktorisches Netzwerk, ein tiefes kontradiktorisches Netzwerk und/oder ein generatives kontradiktorisches Netzwerk sein.

Ein künstliches neuronales Netz (KNN, englisch artificial neural network- ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen. Das künstliche neuronale Netz wurde insbesondere im Vorfeld bereits geeignet für die Bestimmung eines Qualitätsmaßes trainiert.

Die Qualitätseinheit kann dabei von der Magnetresonanzvorrichtung umfasst sein. Beispielsweise kann die Qualitätseinheit von der Recheneinheit der Magnetresonanzvorrichtung umfasst sein. Zudem ist es auch denkbar, dass die Qualitätseinheit von einer Cloud umfasst ist. Hierzu kann die Recheneinheit und/oder die Magnetresonanzvorrichtung eine Datenschnittstelle umfassen, die zu einem Datenaustausch mit der Qualitätseinheit ausgebildet ist.

Das Ermitteln der zumindest einen Bewertungskenngröße erfolgt mittels der Bewertungseinheit. Dabei gehen die zumindest zwei Qualitätsmaße, bevorzugt zumindest drei oder vier Qualitätsmaße, zur Ermittlung der zumindest einen Bewertungskenngröße ein. Neben den Qualitätsmaßen können zudem noch weitere Informationen zur Bestimmung der Bewertungskenngröße von der Bewertungseinheit berücksichtigt werden, wie beispielsweise weitere Bildinformationen und/oder DICOM-Informationen und/oder eine Geräteeigenschaft, insbesondere eine Magnetfeldstärke und/oder eine Gradientenfeldstärke, und/oder eine Gewichtung der einzelnen Qualitätsmaße usw. Zur Ermitteln der zumindest einen Bewertungskenngröße kann die Bewertungseinheit auch ein trainiertes maschinelles Lernverfahren umfassen.

Die Bewertungseinheit kann dabei von der Magnetresonanzvorrichtung umfasst sein. Beispielsweise kann dabei die Bewertungseinheit von der Recheneinheit der Magnetresonanzvorrichtung umfasst sein. Zudem ist es auch denkbar, dass die Bewertungseinheit von einer Cloud umfasst ist. Hierzu kann die Recheneinheit und/oder die Magnetresonanzvorrichtung eine Datenschnittstelle umfassen, die zu einem Datenaustausch mit der Bewertungseinheit ausgebildet ist.

Das Bereitstellen der zumindest einen Bewertungskenngröße erfolgt bevorzugt an ein medizinisches Bedienpersonal, wie beispielsweise eine medizinische-technische Radiologieassistentin. Das Bereitstellen erfolgt dabei bevorzugt mittels der Bewertungseinheit an eine Benutzerschnittstelle, insbesondere eine Ausgabeeinheit der Benutzerschnittstelle, wie beispielsweise einen Monitor und/oder ein Display.

Die Bewertungskenngröße kann beispielsweise eine einfache Einteilung einer Bewertungskennzahl in zumindest zwei Bewertungsklassen der Bildqualität umfassen. Beispielsweise kann dabei die Bewertungskenngröße in einer Bewertungsampel dargestellt werden. Die Farbe Grün kann dabei eine gute Bewertungskenngröße und die Farbe Rot eine schlechte Bewertungskenngröße symbolisieren und/oder darstellen. Zudem kann noch die Farbe Orange für eine mittelmäßige Bewertungskenngröße ausgebeben werden. Alternativ kann auch nur ein "+" oder ein "gut" oder ein "-" oder ein "schlecht" usw. dem Benutzer angezeigt werden.

Das Verfahren zu einem Bereitstellen von zumindest einer Bewertungskenngröße einer Bildqualität von zumindest einem Magnetresonanzbild wird bevorzugt automatisiert bei der Bewertung der Bildqualität, idealerweise bei einer Erfassung von Magnetresonanzdaten während einer Magnetresonanzuntersuchung oder beispielsweise bei einer Erstellung einer Diagnose ausgeführt. Bevorzugt weist hierzu die Magnetresonanzvorrichtung die Recheneinheit zu einer Ausführung des Verfahrens zu einem Bereitstellen zumindest einer Bewertungskenngröße einer Bildqualität von zumindest einem Magnetresonanzbild auf.

Die Recheneinheit umfasst zumindest ein Rechenmodul und/oder einen Prozessor, wobei die Recheneinheit zum Ausführen des erfindungsgemäßen Verfahrens zu einem Bereitstellen zumindest einer Bewertungskenngröße einer Bildqualität von zumindest einem Magnetresonanzbild ausgebildet ist. So ist insbesondere die Recheneinheit dazu ausgebildet, computerlesbare Instruktionen auszuführen, um das erfindungsgemäße Verfahren auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen, um das erfindungsgemäßes Verfahren zu einem Bereitstellen zumindest einer Bewertungskenngröße einer Bildqualität von zumindest einem Magnetresonanzbild auszuführen.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Erfindung weist den Vorteil auf, dass eine schnelle und robuste Bewertung einer Bildqualität während einer Magnetresonanzuntersuchung für ein medizinisches Bedienpersonal bereitgestellt werden kann. Insbesondere kann derart das medizinische Bedienpersonal ein direktes Feedback bezüglich der Bildqualität während einer Magnetresonanzuntersuchung erhalten. Weiterhin kann derart das medizinische Bedienpersonal vorteilhaft unterstützt werden und dabei manuelle und/oder subjektive Fehler bei der Bewertung der Bildqualität reduziert und/oder verhindert werden und eine objektive Bewertung der Bildqualität erfolgen. Insbesondere können die einzelnen Qualitätsmaße unterschiedlich Qualitätsprobleme bei Magnetresonanzbildern abdecken, so dass eine Fehleranfälligkeit des Verfahrens reduziert wird.

Des Weiteren kann ein medizinisches Bedienpersonal anhand der Bewertungskenngröße bei Vorliegen einer mangelnden Bildqualität sofortige Abhilfemaßnahmen zur Verbesserung der Bildqualität ergreifen, wie beispielsweise eine erneute Messung durchführen und/oder Beruhigungsmaßnahmen zur Beruhigung des Patienten, wenn dieser sich während der Messung bewegt hat, einleiten und/oder eine alternativen Messsequenz für die erneute Messung auswählen usw. Derart kann auch eine Diagnose anhand der erfassten Magnetresonanzdaten, insbesondre Magnetresonanzbilder, verbessert werden, da nunmehr Magnetresonanzbilder mit einer hohen Bildqualität vorliegen.

In einer vorteilhaften Weiterbildung des erfindungsgemäßen Verfahrens kann es vorgesehen sein, dass das Bereitstellen der zumindest einen Bewertungskenngröße einer Bildqualität von zumindest einem Magnetresonanzbild während einer Magnetresonanzuntersuchung durchgeführt wird. Bevorzugt werden direkt bei der Bilddatenerfassung, insbesondere der Erfassung von Magnetresonanzdaten, Magnetresonanzbilder rekonstruiert und für diese Magnetresonanzbilder direkt zumindest eine Bewertungskenngröße einer Bildqualität ermittelt und bereitgestellt. Derart kann eine vorteilhafte Unterstützung, bevorzugt eine automatisierte Unterstützung, des Benutzers bei der Bewertung und/oder einer Einschätzung einer Bildqualität während einer Magnetresonanzuntersuchung erreicht werden.

In einer vorteilhaften Weiterbildung des erfindungsgemäßen Verfahrens kann es vorgesehen sein, dass zumindest eines der zwei oder mehr Qualitätsmodule ein trainiertes maschinelles Lernverfahren umfasst, wobei das trainierte maschinelle Lernverfahren dazu trainiert ist, ein Qualitätsmaß des zu bewertenden Magnetresonanzbilds durch Vergleich mit zumindest einem Referenzbild zu bestimmen. Bevorzugt umfasst das Referenzbild eine ideale Kopie des Magnetresonanzbildes ohne jegliche Qualitätseinschränkungen.

Das maschinelle Lernverfahren wurde insbesondere im Vorfeld bereits geeignet für die Bestimmung eines Qualitätsmaßes trainiert. Für das Training des maschinellen Lernverfahrens wurden dabei insbesondere Trainingsbilddatensätze verwendet, bei denen Magnetresonanzbilder mit zumindest einem Referenzbild hinsichtlich des zu bestimmenden Qualitätsmaßes verglichen wurden und eine Abweichung und/oder ein Unterschied des Magnetresonanzbilds zu dem zumindest einen Referenzbild das entsprechende Qualitätsmaß festlegt. Weisen zwei oder mehr der Qualitätsmodule ein maschinelles Lernverfahren auf, dann sind bevorzugt die maschinellen Lernverfahren auf die Bestimmung des jeweiligen Qualitätsmaßes trainiert. Die medizinischen Trainingsdatensätze sind dabei typischerweise von verschiedenen Trainings-Personen und/oder Trainings-Patienten akquiriert worden. Vorzugsweise umfassen dabei die medizinischen Trainingsdatensätze Datensätze von unterschiedlichen Körperbereichen, so dass für Magnetresonanzuntersuchungen von unterschiedlichen Körperbereiche eine bevorzugt automatisierte Bewertung der Bildqualität erfolgen kann.

Derart kann eine schnelle und robuste Bewertung einer Bildqualität während einer Magnetresonanzuntersuchung für ein medizinisches Bedienpersonal bereitgestellt werden. Insbesondere kann derart das medizinische Bedienpersonal vorteilhaft unterstütz werden und dabei manuelle und/oder subjektive Fehler bei der Bewertung der Bildqualität reduziert und/oder verhindert werden.

In einer vorteilhaften Weiterbildung des erfindungsgemäßen Verfahrens kann es vorgesehen sein, dass zumindest eines der zwei oder mehr Qualitätsmodule ein trainiertes tiefes Lernverfahren umfasst, wobei das trainierte tiefe Lernverfahren dazu trainiert ist, ein Qualitätsmaß des zu bewertenden Magnetresonanzbilds ohne Vergleich mit einem Referenzbild zu bestimmen.

Das tiefe Lernverfahren (engl.: deep learning) umfasst eine Methode des maschinellen Lernens, die künstliche neuronale Netze mit zahlreichen Zwischenschichten zwischen Eingabeschicht und Ausgabeschicht einsetzt und dadurch eine umfangreiche innere Struktur herausbildet. Für das Training des tiefen Lernverfahrens wurden dabei insbesondere Trainingsbilddatensätze verwendet, bei denen Magnetresonanzbilder mit unterschiedlichen Bildqualitäten das entsprechende Qualitätsmaß zugeordnet sind. Die Trainingsdaten der tiefen Lernverfahren können zudem auch ein Referenzbild umfassen. Weisen zwei oder mehr der Qualitätsmodule ein tiefes Lernverfahren auf, dann sind bevorzugt die tiefen Lernverfahren auf die Bestimmung des jeweiligen Qualitätsmaßes trainiert. Insbesondere können neuronaler Netze (mit oder ohne Zuhilfenahme von Referenzbildern) trainiert werden zur Testzeit auch ohne Vorhandensein von Referenzbildern Bildqualitätsmaße vorherzusagen, welche für die direkte Berechnung jeweils ein perfektes Referenzbild benötigen würden. So kann zum Beispiel ein neuronales Netz trainiert werden die MSSIM vorherzusagen, ohne zum Testzeitpunkt ein Referenzbild zu benötigen. Dies ist ein signifikanter, technischer und strategischer Vorteil, da in der klinischen Realität Referenzbilder nicht zur Verfügung stehen und diese Methode dennoch die Berücksichtigung von eine Großzahl von Qualitätsmaßen ermöglicht, welche für die direkt Berechnung ein Referenzbild benötigen würden.

Die medizinischen Trainingsdatensätze sind dabei typischerweise von verschiedenen Trainings-Personen und/oder Trainings-Patienten akquiriert worden. Vorzugsweise umfassen dabei die medizinischen Trainingsdatensätze Datensätze von unterschiedlichen Körperbereichen, so dass für Magnetresonanzuntersuchungen von unterschiedlichen Körperbereiche eine bevorzugt automatisierte Bewertung der Bildqualität erfolgen kann. Das Qualitätsmaß aus den tiefen Lernverfahren kann dabei eine prognostizierte Punktezahl umfassen, die auf Aufgaben hinsichtlich einer Bilderkennung und/oder einer Bildqualität trainiert sind.

Diese Ausgestaltung weist den Vorteil auf, dass eine robuste Bestimmung eines Qualitätsmaßes erfolgen kann. Zudem weisen sich tiefe Lernverfahren dadurch aus, dass mit einem kleinen Trainingsdatensatz das Verfahren trainiert werden kann und damit auch eine schnelle Anpassung an Änderungen, beispielsweise Änderungen der Hardwarebedingungen und/oder eine Auswahl eines anderen Körperbereichs usw., erreicht werden kann. Zudem können derart unterschiedliche, insbesondere individuelle, Qualitätsprobleme bei der Ermittlung des jeweiligen Qualitätsmaßes vorteilhaft berücksichtigt werden.

In einer vorteilhaften Weiterbildung des erfindungsgemäßen Verfahrens kann es vorgesehen sein, dass die Bewertungseinheit ein maschinelles Lernverfahren zur Ermittlung der zumindest einen Bewertungskenngröße aus den zumindest zwei Qualitätsmaßen umfasst. Das maschinelle Lernverfahren kann dabei eine Support Vector Machine (SVM), eine Support Vector Regression (SVR), einen Entscheidungsbaum (decision tree), eine lineare Regression, einen Linear Forest usw. umfassen. Zudem kann das maschinelle Lernverfahren auch ein tiefes Lernverfahren, wie beispielsweise ein Convolutional Neural Network (CNN) oder ein Fully Connected Network, umfassen.

Vorzugsweise ist das maschinelle Lernverfahren derart trainiert, dass aus einer Kombination von mehreren, voneinander unabhängigen, Qualitätsmaßen eine Bewertungskenngröße zur Bewertung der Bildqualität des zumindest einen Magnetresonanzbildes ermittelt werden kann. Zudem kann das maschinelle Lernverfahren auch an einen Anwender angepasst trainiert werden, um beispielsweise Qualitätskriterien eines Anwenders bei der Bewertung der Bildqualität des zumindest einen Magnetresonanzbildes zu berücksichtigen. Beispielsweise können derart individuelle Qualitätskriterien, wie beispielsweise ein Rauschanteil in einem Magnetresonanzbild, von einem Benutzer definiert und/oder vorgegeben werden und in einer Trainingsphase des maschinellen Lernverfahrens berücksichtigt werden. Das maschinelle Lernverfahren zur Bestimmung der Bewertungskenngröße kann zudem auf Grundlage von Bewertungen von Experten, beispielsweise von Ärzten, trainiert sein und damit einen beste Übereinstimmung zwischen einer Expertenbewertung und den bereitgestellten Qualitätsmaßen bereitgestellt werden.

In einer vorteilhaften Weiterbildung des erfindungsgemäßen Verfahrens kann es vorgesehen sein, dass die Bewertungseinheit zur Ermittlung der zumindest einen Bewertungskenngröße aus den zumindest zwei Qualitätsmaßen zumindest eine statistische Methode verwendet. Die statistische Methode kann dabei einen arithmetischen Mittelwert und/oder einen Median und/oder eine Standardabweichung der Qualitätsmaße und/oder ein Maximum und/oder ein Minimum usw. umfassen. Derart kann auf einfache Art und Weise die Bewertungskenngröße ermittelt werden.

In einer vorteilhaften Weiterbildung des erfindungsgemäßen Verfahrens kann es vorgesehen sein, dass zur Ermittlung der zumindest einen Bewertungskenngröße des zumindest einen Magnetresonanzbilds weitere Bildparameter und/oder DICOM-Parameter berücksichtigt werden. Die weiteren Bildparameter und/oder DICOM-Parameter können beispielsweise eine Akquisitions-Zeit und/oder Pixelgröße und/oder eine Matrixgröße und/oder eine Bildauflösung und/oder einen Sequenzparameter und/oder ein Geschlecht des Patienten und/oder ein Alter des Patienten und/oder eine Magnetfeldstärke und/oder eine Gradientenfeldstärke usw. umfassen. Derart kann eine zusätzliche Information, insbesondere eine kontextbezogene Information, für die einzelnen Qualitätsmaße vorliegen, die bei der Ermittlung der Bewertungskenngröße aus den einzelnen Qualitätsmaßen berücksichtigt werden. Beispielsweise kann anhand der zusätzlichen Information eine Gewichtung der einzelnen Qualitätsmaße bei der Ermittlung der Bewertungskenngröße beeinflusst werden. So kann beispielsweise ein Rauschverhalten in Abhängigkeit von einer Pixelgröße und/oder einer Sequenzeigenschaft gewichtet werden.

In einer vorteilhaften Weiterbildung des erfindungsgemäßen Verfahrens kann es vorgesehen sein, dass in der Bewertungseinheit zur Ermittlung der zumindest einen Bewertungskenngrö-βe eine Gewichtung der einzelnen ermittelten Qualitätsmaße erfolgt. Derart können unterschiedliche Qualitätsmaße mit einer unterschiedlichen Gewichtung berücksichtigt werden. Dabei kann es auch vorgesehen sein, dass eine Gewichtung der einzelnen Qualitätsmaße zumindest teilweise durch einen Benutzer, insbesondere das medizinische Bedienpersonal, erfolgen kann. Beispielsweise kann dabei ein Benutzer über eine Benutzerschnittstelle eine individuelle Gewichtung der einzelnen Qualitätsmaße bei der Ermittlung der zumindest einen Bewertungskenngröße festlegen. Hierdurch kann der Vorteil erreicht werden, dass eine einfache und schnelle, insbesondere individuelle, Anpassung bei der Ermittlung der zumindest einen Bewertungskennzahl erreicht werden kann. Zudem können derart auch einzelne bereitgestellte Qualitätsmaße, die beispielsweise bei unterschiedlichen Sequenzen eine unterschiedlich gute Übereinstimmung mit einer Expertenbewertung aufweisen, für die unterschiedlichen Sequenzen unterschiedlich gewichtet werden. Beispielsweise können auch diejenigen Qualitätsmodule und/oder Qualitätsmaße mit der besten Übereinstimmung mit einer Expertenbewertung für die jeweilige Sequenz hoch gewichtet werden. Hingegen können diejenigen Qualitätsmodule und/oder Qualitätsmaße mit der geringsten Übereinstimmung mit einer Expertenbewertung für die jeweilige Sequenz niedrig gewichtet werden.

In einer vorteilhaften Weiterbildung des erfindungsgemäßen Verfahrens kann es vorgesehen sein, dass in der Bewertungseinheit zur Ermittlung der zumindest einen Bewertungskenngrö-βe die zwei oder mehr Qualitätsmaße zur Klassifizierung der einzelnen Qualitätsmaße mit jeweils zumindest einem Eingangsschwellwert verglichen werden. Die Klassifizierung der einzelnen Qualitätsmaße kann beispielsweise jeweils zwei Klassen umfassen, wobei die zwei Klassen in "gut" und "schlecht" unterteilt sind. Zudem kann die Klassifizierung auch mehr als zwei Klassen umfassen. Dabei können für jedes der zwei oder mehr Qualitätsmaße unterschiedliche Eingangsschwellwerte zur Klassifizierung der einzelnen Qualitätsmaße vorliegen und/oder verwendet werden. Die Klassifizierung der zwei oder mehr Qualitätsmaße weist den Vorteil auf, dass nicht der absolute Wert des ermittelten Qualitätsmaßes in die Ermittlung der zumindest einen Bewertungskenngröße einfließt, sondern eine Art Normierung und/oder Wertung des Qualitätsmaßes. Beispielsweise kann ein Wert für das Qualitätsmaß für das Modul MSSIM, der umgedreht angegeben wird (1-MSSIM) für eine Klassifikation "gut" nicht unter 0,933 liegen, während das Qualitätsmaß für das Modul BRISQUE bei dem Wert bereits bei 0,598 einen derartige Klassifikation erreicht. Derart kann eine unerwünschte Fehlinterpretation einzelner Qualitätsmaße verteilhaft verhindert werden.

In einer vorteilhaften Weiterbildung des erfindungsgemäßen Verfahrens kann es vorgesehen sein, dass die zumindest eine Bewertungskenngröße eine Klassifizierung der Bildqualität in zumindest zwei Qualitätsklassen umfasst, wobei eine Auswahl einer Qualitätsklasse in Abhängigkeit von einer ermittelten Bewertungszahl erfolgt. Die Bewertungszahl umfasst bevorzugt eine Zahl zwischen 0 und 1, die umso kleiner ist, je höher auch eine Bildqualität der Magnetresonanzbilddaten ist, wobei die Bewertungszahl anhand der zumindest zwei Qualitätsmaße und weiterer Parameter von der Bewertungseinheit ermittelt wurde. Die zumindest zwei Qualitätsklassen umfassen dabei eine Qualitätsklasse mit einer hohen Bildqualität und eine Qualitätsklasse mit einer niedrigen und/oder unzureichenden Bildqualität. Vorzugsweise wird hierbei die ermittelte Bewertungszahl mit zumindest einem Schwellwert verglichen, um eine Eingruppierung und/oder eine Klassifikation der Bewertungskennzahl vorzunehmen. Bevorzugt umfasst der Schwellwert eine Grenze zwischen zwei Qualitätsklassen.

Die Qualitätsklasse mit der hohen Bildqualität kann dabei ein Ausgabeelement, insbesondere ein visuelles Ausgabeelement umfassen, dass mittels der Benutzerschnittstelle an den Benutzer ausgebbar ist. Das visuelle Ausgabeelement der Qualitätsklasse mit der hohen Bildqualität kann dabei ein "gut" und/oder "+" und/oder einen lachenden Smiley und/oder einem grünen Punkt usw. umfassen.

Die Qualitätsklasse mit der niedrigen und/oder unzureichenden Bildqualität kann dabei ein Ausgabeelement, insbesondere ein visuelles Ausgabeelement umfassen, dass mittels der Benutzerschnittstelle an den Benutzer ausgebbar ist. Das visuelle Ausgabeelement der Qualitätsklasse mit der niedrigen und/oder unzureichenden Bildqualität kann dabei ein "schlecht" und/oder "-" und/oder einen weinenden Smiley und/oder einen roten Punkt usw. umfassen.

Zudem kann die Bewertungskenngröße auch eine Klassifizierung der Bildqualität in drei Qualitätsklassen umfassen, wobei die Klassifizierung eine zusätzliche Qualitätsklasse mit einer mittleren Qualität vorsieht. Die Qualitätsklasse mit der mittleren Bildqualität kann dabei ein Ausgabeelement, insbesondere ein visuelles Ausgabeelement umfassen, dass mittels der Benutzerschnittstelle an den Benutzer ausgebbar ist. Das visuelle Ausgabeelement der Qualitätsklasse mit mittleren Bildqualität kann dabei ein "mittel" und/oder einen neutral aussehenden Smiley und/oder einen orangen oder gelben Punkt usw. umfassen.

Diese Ausgestaltung der Erfindung weist den Vorteil auf, dass eine einfache und direkte Erfassung einer Qualität der bereitgestellten Magnetresonanzbilder für einen Benutzer erreicht werden kann. Insbesondere kann während einer Magnetresonanzmessung eine direkte Ausgabe der Bewertungskenngröße zur Bewertung der Bildqualität erfolgen.

In einer vorteilhaften Weiterbildung des erfindungsgemäßen Verfahrens kann es vorgesehen sein, dass die Bewertungskenngröße die ermittelte Bewertungszahl umfasst. Die ermittelte Bewertungszahl kann dabei zusätzlich zu einer Anzeige einer Qualitätsklasse der Bewertungskenngröße ausgegeben und/oder angezeigt werden. Durch die Ausgabe der Bewertungszahl kann ein Benutzer zudem eine detaillierte Beurteilung der Bildqualität erhalten. Beispielsweise kann ein Benutzer durch die Bewertungszahl erkennen, dass zwar die niedrige Qualitätsklasse vorliegt, jedoch die Bewertungszahl sehr nah am Schwellwert zur nächsthöheren Qualitätsklasse liegt. Dies ermöglicht den Benutzer eine individuelle Einstufung der Bildqualität der Magnetresonanzbilddaten.

In einer vorteilhaften Weiterbildung des erfindungsgemäßen Verfahrens kann es vorgesehen sein, dass die zumindest eine Bewertungskenngröße an einen Benutzer ausgegeben wird. Vorzugsweise erfolgt die Ausgabe visuell mittels einer Darstellungseinheit, wie beispielweise einen Monitor und/oder ein Display, einer Benutzerschnittstelle der Magnetresonanzvorrichtung. Hierdurch kann eine einfache und direkte Erfassung einer Qualität der bereitgestellten Magnetresonanzbilder erreicht werden. Insbesondere kann dem Benutzer, beispielsweise ein Arzt, direkt bei einer Magnetresonanzdatenerfassung die Bewertungskenngröße ausgegeben werden, so dass dieser direkt auf eine schlechte Bildqualität reagieren kann.
Die Erfindung geht des Weiteren aus von einem System mit einer Magnetresonanzvorrichtung, einer Qualitätseinheit und einer Bewertungseinheit, wobei das System zu einem Ausführen eines Verfahrens zu einem Bereitstellen von zumindest einer Bewertungskenngröße einer Bildqualität von zumindest einem Magnetresonanzbild ausgebildet ist.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst dabei eine Magneteinheit und/oder eine Scannereinheit mit einem Grundmagneten, einer Gradientenspuleneinheit und einer Hochfrequenzantenneneinheit.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden.

Für eine Magnetresonanzuntersuchung wird der Patient, insbesondere der zu untersuchende Bereich des Patienten, innerhalb des Patientenaufnahmebereichs der Magnetresonanzvorrichtung positioniert. Der Patientenaufnahmebereich ist dabei zumindest teilweise von der Magneteinheit umgeben, insbesondere zylinderförmig von der Magneteinheit umgeben. Innerhalb des Patientenaufnahmebereichs ist bevorzugt ein Field of View (FOV) und ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Mittels der Magnetresonanzvorrichtung werden Magnetresonanzbilddaten, insbesondere Magnetresonanzbilder, für eine Bewertung der Bildqualität bereitgestellt.

Die Qualitätseinheit ist zu einem Bestimmen von zumindest zwei unterschiedlichen Qualitätsmaßen des zumindest einen Magnetresonanzbilds ausgebildet. Hierzu umfasst die Qualitätseinheit zwei oder mehr Qualitätsmodule, wobei jedes der zwei oder mehr Qualitätsmodule zur Bestimmung eines der zwei oder mehr unterschiedlichen Qualitätsmaße ausgebildet ist. Zudem ist die Qualitätseinheit auch zu einem Bereitstellen der zwei oder mehr Qualitätsmaße an eine Bewertungseinheit ausgebildet.

Die Bewertungseinheit ist zu einem Ermitteln der zumindest einer Bewertungskenngröße ausgebildet. Dabei wird die zumindest eine Bewertungskenngröße anhand der zwei oder mehr unterschiedlichen Qualitätsmaße ermittelt.

Die Qualitätseinheit und/oder die Bewertungseinheit können dabei von der Magnetresonanzvorrichtung umfasst sein. Dabei können die Qualitätseinheit und/oder die Bewertungseinheit beispielsweise von einer Recheneinheit der Magnetresonanzvorrichtung umfasst sein. Alternativ hierzu kann die Qualitätseinheit und/oder die Bewertungseinheit auch von einer Cloud umfasst sein. Hierzu kann die Recheneinheit und/oder die Magnetresonanzvorrichtung eine Datenschnittstelle umfassen, die zu einem Datenaustausch mit der Qualitätseinheit und/oder der Bewertungseinheit ausgebildet ist.

Des Weiteren umfasst die Magnetresonanzvorrichtung eine Benutzerschnittstelle, die zu einer Ausgabe der zumindest einen Bewertungskenngröße ausgebildet ist.

Das erfindungsgemäße System weist den Vorteil auf, dass eine schnelle und robuste Bewertung einer Bildqualität während einer Magnetresonanzuntersuchung für ein medizinisches Bedienpersonal bereitgestellt werden kann. Insbesondere kann derart das medizinische Bedienpersonal ein direkte Feedback bezüglich der Bildqualität während einer Magnetresonanzuntersuchung erhalten. Weiterhin kann derart das medizinische Bedienpersonal vorteilhaft unterstützt werden und dabei manuelle und/oder subjektive Fehler bei der Bewertung der Bildqualität reduziert und/oder verhindert werden und eine objektive Bewertung der Bildqualität erfolgen. Insbesondere können die einzelnen Qualitätsmaße unterschiedlich Qualitätsprobleme bei Magnetresonanzbildern abdecken, so dass eine Fehleranfälligkeit des Verfahrens reduziert wird.

Die Vorteile des erfindungsgemäßen Systems entsprechen im Wesentlichen den Vorteilen der erfindungsgemäßen Verfahrens zu einem Bereitstellen von zumindest einer Bewertungskenngröße einer Bildqualität von zumindest einem Magnetresonanzbild, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Recheneinheit ladbar ist, mit Programmmitteln, um ein Verfahren zu einem Bereitstellen von zumindest einer Bewertungskenngröße einer Bildqualität von zumindest einem Magnetresonanzbild auszuführen, wenn das Programm in der Recheneinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch kompiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung in einer schematischen Darstellung,
- Fig. 2: das erfindungsgemäße Verfahren und
- Fig. 3: ein Ablaufdiagramm des Verfahrens.

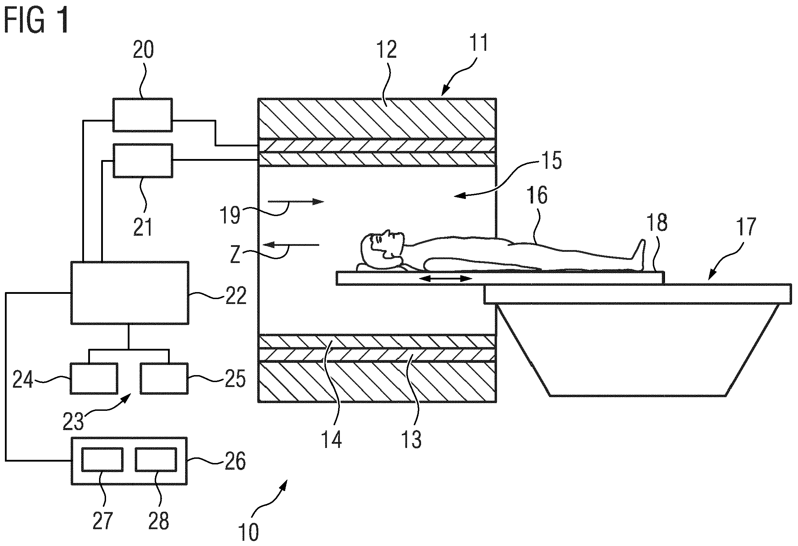

In der Fig. 1 ist eine Magnetresonanzvorrichtung 10 schematisch dargestellt. Die Magnetresonanzvorrichtung 10 umfasst eine Magneteinheit 11 mit einem Grundmagneten 12, einer Gradientenspuleneinheit 13 und einer Hochfrequenzantenneneinheit 14. Zudem weist die Magnetresonanzvorrichtung 10 einen Patientenaufnahmebereich 15 auf zu einer Aufnahme eines Patienten 16. Der Patientenaufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 15 jederzeit denkbar.

Für eine Positionierung des Patienten 16, insbesondere eines zu untersuchenden Bereichs des Patienten 16, innerhalb des Patientenaufnahmebereichs 15 weist die Magnetresonanzvorrichtung 10 eine Patientenlagerungsvorrichtung 17 auf. Die Patientenlagerungsvorrichtung 17 weist einen bewegbaren Patiententisch 18 auf. Der Patiententisch 18 ist für eine Positionierung des Patienten 16, insbesondere des zu untersuchenden Bereichs des Patienten 16, bewegbar innerhalb des Patientenaufnahmebereichs 15 ausgebildet.

Der Grundmagnet 12 der Magneteinheit 11 ist zu einem Erzeugen eines starken und insbesondere konstanten Grundmagnetfelds 19 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 20 der Magnetresonanzvorrichtung 10 gesteuert. Die Hochfrequenzantenneneinheit 14 der Magneteinheit 10 ist zu einer Anregung einer Polarisation, die sich in dem von dem Grundmagneten 12 erzeugten Grundmagnetfeld 19 einstellt, ausgebildet. Die Hochfrequenzantenneneinheit 14 wird von einer Hochfrequenzantennensteuereinheit 21 der Magnetresonanzvorrichtung 10 gesteuert und strahlt hochfrequente Magnetresonanzsequenzen in den Patientenaufnahmebereich 15 der Magnetresonanzvorrichtung 10 ein.

Zu einer Steuerung des Grundmagneten 12, der Gradientensteuereinheit 20 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Darstellungseinheit und/oder Ausgabeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Im vorliegenden Ausführungsbeispiel weist die Magnetresonanzvorrichtung 10 eine Recheneinheit 26 auf, die eine Qualitätseinheit 27 und eine Bewertungseinheit 28 umfasst. In einer alternativen Ausgestaltung der Magnetresonanzvorrichtung 10 kann die Recheneinheit 26, insbesondere die Qualitätseinheit 27 und die Bewertungseinheit 28, auch separat zur Magnetresonanzvorrichtung 10 ausgebildet sein. Dabei kann die Recheneinheit 26, insbesondere die Qualitätseinheit 27 und die Bewertungseinheit 28, auch in einer Cloud angeordnet sein.

Die dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

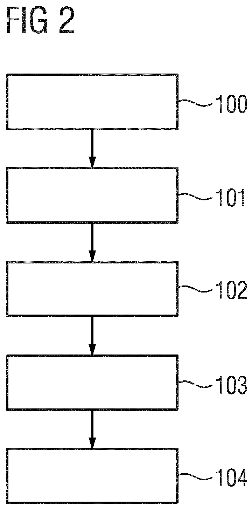

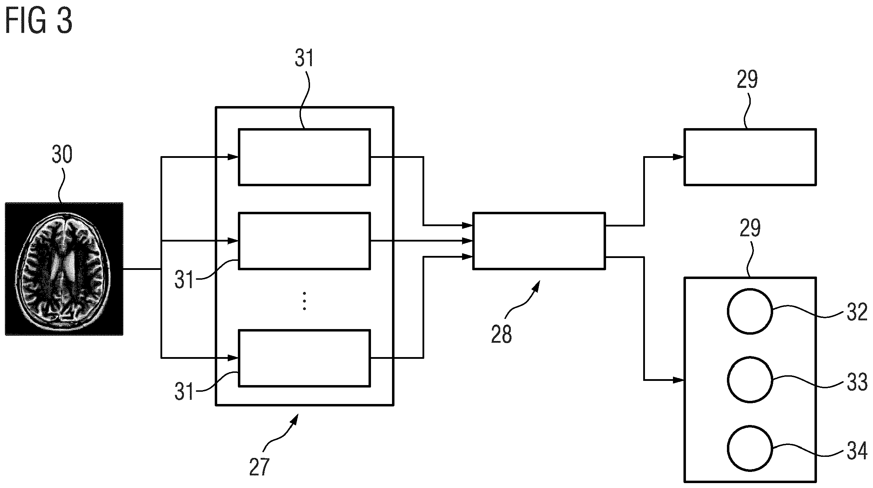

In den Fig. 2 und 3 ist das erfindungsgemäße Verfahren zu einem Bereitstellen von zumindest einer Bewertungskenngröße 29 einer Bildqualität von zumindest einem Magnetresonanzbild 30 dargestellt. Das Verfahren wird dabei automatisch von der Recheneinheit 26 ausgeführt, die hierzu eine erforderliche Software und/oder Computerprogramme aufweist.

In einem ersten Verfahrensschritt 100 erfolgt eine Bereitstellen des zumindest einen Magnetresonanzbilds 30. Bevorzugt werden hierbei die während der Magnetresonanzuntersuchung erfassten Magnetresonanzbilder 30 direkt für ein Bereitstellen der zumindest einen Bewertungskenngröße bereitgestellt. Das Bereitstellen erfolgt hierbei mittels der Recheneinheit 26 an die Qualitätseinheit 27.

In einem daran anschließenden zweiten Verfahrensschritt 101 erfolgt ein Bestimmen von zumindest zwei unterschiedlichen Qualitätsmaßen des zumindest einen Magnetresonanzbilds 30 mittels der Qualitätseinheit 27. Hierzu umfasst die Qualitätseinheit 27 zwei oder mehr Qualitätsmodule 31 zur Bestimmung der zwei oder mehr unterschiedlichen Qualitätsmaße. Die zwei oder mehr Qualitätsmodule 31 sind dabei unabhängig zueinander ausgebildet. Auch sind die zwei oder mehr Qualitätsma-βe unabhängig zueinander. Bevorzugt werden für ein Bereitstellen der zumindest einen Bewertungskenngröße drei oder Qualitätsmaße mittels drei oder vier Qualitätsmodule 31 ermittelt.

Die unterschiedlichen Qualitätsmodule 31 können beispielsweise unterschiedliche Standard-Bildqualitätseigenschaften umfassen. Dabei können die Qualitätsmodule 31 die folgenden Qualitätseigenschaften bei der Bestimmung des jeweiligen Qualitätsmaßes berücksichtigen:
- Normalisiertes Quadratisches Mittel (normalized root mean square - NRMS)
- Spitzen-Signal-Rausch-Verhältnis (Peak signal-to-noise ratio - PSNR)
- DCT (discrete cosine transform) subbands similarity (DSS)
- Gradient magnitude similarity deviation (GMSD)
- Haar wavelet-based Perceptual similarity index (HaarPSI)
- Mean deviation similarity index (MDSI)
- Mean structural similarity index measure (MSSIM)
- Multi-scale structural similarity index measure (MSSSIM)
- Visual information fidelity (VIF)
- Visual saliency-based index (VSI)
- Deep image structure and texture similarity (DISTS)
- Learned perceptual image patch similarity (LPIPS)
- Perceptual image error metric (PieAPP)
- Total variation (TV)
- Blind referenceless image spatial quality evaluator (BRISQUE)
- Natural image quality evaluator (NIQE)

Zudem können die zwei oder mehr Qualitätsmodule 31 jeweils ein trainiertes maschinelles Lernverfahren umfassen, wobei die einzelnen trainierten maschinellen Lernverfahren dazu trainiert sind, ein Qualitätsmaß des zu bewertenden Magnetresonanzbilds 30 durch Vergleich mit zumindest einem Referenzbild zu bestimmen. Das maschinelle Lernverfahren wurde insbesondere im Vorfeld bereits geeignet für die Bestimmung eines Qualitätsmaßes trainiert. Für das Training des maschinellen Lernverfahrens zur Vorhersage/Ermittlung von Qualitätsmaßen, welche auf der Basis von Referenzbildern definiert sind, wurden dabei insbesondere Trainingsbilddatensätze verwendet, bei denen Magnetresonanzbilder mit zumindest einem Referenzbild hinsichtlich des zu bestimmenden Qualitätsmaßes verglichen wurden und eine Abweichung und/oder ein Unterschied des Magnetresonanzbilds zu dem zumindest einen Referenzbild das entsprechende Qualitätsmaß festlegt.

Alternativ hierzu können die zwei oder mehr Qualitätsmodule 31 auch ein trainiertes tiefes Lernverfahren umfassen, wobei die einzelnen trainierten tiefen Lernverfahren dazu trainiert sind, ein Qualitätsmaß des zu bewertenden Magnetresonanzbilds ohne Vergleich mit einem Referenzbild zu bestimmen. Für das Training des tiefen Lernverfahrens wurden dabei insbesondere Trainingsbilddatensätze verwendet, bei denen Magnetresonanzbilder mit unterschiedlichen Bildqualitäten unterschiedlichen Qualitätsmaße zugeordnet sind. Die Trainingsdaten der tiefen Lernverfahren können zudem auch ein Referenzbild umfassen. Vorzugsweise umfassen dabei die medizinischen Trainingsdatensätze Datensätze von unterschiedlichen Körperbereichen, so dass für unterschiedliche Körperbereiche eine bevorzugt automatisierte Bewertung der Bildqualität erfolgen kann. Das Qualitätsmaß aus den tiefe Lernverfahren kann dabei eine prognostizierte Punktezahl umfassen, die auf Aufgaben hinsichtlich einer Bilderkennung und/oder einer Bildqualität trainiert wurden.

Mittels der Qualitätseinheit 27 erfolgt zudem in diesem zweiten Verfahrensschritt 101 ein Bereitstellen zwei oder mehr Qualitätsmaße an die Bewertungseinheit 28.

In einem daran anschließenden dritten Verfahrensschritt 102 erfolgt ein Ermitteln der zumindest einen Bewertungskenngröße 29 mittels der Bewertungseinheit 28, wobei die zumindest eine Bewertungskenngröße 29 anhand der zwei oder mehr unterschiedlichen Qualitätsmaße ermittelt wird.

Für die Ermittlung der zumindest einen Bewertungskenngröße 29 aus den zumindest zwei unterschiedlichen Qualitätsmaßen umfasst die Bewertungseinheit 28 bevorzugt ein maschinelles Lernverfahren. Das maschinelle Lernverfahren kann dabei eine Support Vector Machine (SVM), eine Support Vector Regression (SVR), einen Entscheidungsbaum (decision tree), eine lineare Regression, einen Linear Forest usw. umfassen. Zudem kann das maschinelle Lernverfahren auch ein tiefes Lernverfahren, wie beispielsweise ein Convolutional Neural Network (CNN) oder ein Fully Connected Network, umfassen.

Das maschinelle Lernverfahren kann dabei derart trainiert sein, dass aus einer Kombination von mehreren, voneinander unabhängigen Qualitätsmaßen eine Bewertungskenngröße 29 zur Bewertung der Bildqualität des zumindest einen Magnetresonanzbildes 30 ermittelt werden kann. Zudem kann das maschinelle Lernverfahren auch an einen Anwender angepasst trainiert werden, um beispielsweise Qualitätskriterien eines Anwenders bei der Bewertung der Bildqualität des zumindest einen Magnetresonanzbildes 30 zu berücksichtigen. Das maschinelle Lernverfahren zur Bestimmung der Bewertungskenngröße kann zudem auf Grundlage von Bewertungen von Experten, beispielsweise von Ärzten, trainiert sein und damit einen beste Übereinstimmung zwischen einer Expertenbewertung und den bereitgestellten Qualitätsmaßen bereitgestellt werden.

Zudem kann die Bewertungseinheit 28 zur Ermittlung der zumindest einen Bewertungskenngröße aus den zumindest zwei unterschiedlichen Qualitätsmaßen zumindest eine statistische Methode umfassen und/oder verwenden. Die statistische Methode kann dabei einen arithmetischen Mittelwert und/oder einen Median und/oder eine Standardabweichung der Qualitätsmaße und/oder ein Maximum und/oder ein Minimum umfassen.

Des Weiteren können in diesem dritten Verfahrensschritt 102 weitere Bildparameter und/oder DICOM-Parameter bei der Ermittlung der zumindest Bewertungskenngröße 29 berücksichtigt werden, wie beispielsweise eine Akquisitions-Zeit und/oder Pixelgröße und/oder eine Matrixgröße und/oder eine Bildauflösung und/oder ein Sequenzparameter und/oder ein Geschlecht des Patienten und/oder ein Alter des Patienten und/oder eine Magnetfeldstärke und/oder eine Gradientenfeldstärke usw.

Weiterhin kann in diesem dritten Verfahrensschritt 102 eine Gewichtung der einzelnen Qualitätsmaße bei der Ermittlung der zumindest Bewertungskenngröße 29 erfolgen. Zudem kann die Gewichtung der einzelnen Qualitätsmaße auch von einem Benutzer festgelegt werden, beispielsweise mittels der Eingabeeinheit 25 der Benutzerschnittstelle 23, um eine individuelle Anpassung und/oder individuelle Einstellungen bei der Ermittlung der zumindest Bewertungskenngröße 29 zu berücksichtigen.

Des Weiteren erfolgt in diesem dritten Verfahrensschritt 102 von der Bewertungseinheit 28 zur Ermittlung der zumindest einen Bewertungskenngröße 29, dass die zwei oder mehr Qualitätsmaße zur Klassifizierung der einzelnen Qualitätsmaße mit jeweils zumindest einem Eingangsschwellwert verglichen werden. Der Eingangsschwellwert kann dabei individuell an das jeweilige Qualitätsmaß angepasst sein.

Die ermittelte Bewertungskenngröße 29 wird zudem in diesem dritten Verfahrensschritt 102 klassifiziert, wobei eine Klassifizierung der Bildqualität zumindest zwei Qualitätsklassen 32, 33, 34 umfasst. Eine Auswahl einer Qualitätsklasse 32, 33, 34 erfolgt dabei in Abhängigkeit von einer ermittelten Bewertungszahl, die mit einem Schwellwert verglichen wird. Die zumindest zwei Qualitätsklassen 32, 33, 34 der Bewertungskenngröße 29 umfassen dabei eine Qualitätsklasse 32 mit einer hohen Bildqualität und eine Qualitätsklasse 34 mit einer niedrigen und/oder unzureichenden Bildqualität. Zudem kann die Bewertungskenngröße 29 auch eine Klassifizierung der Bildqualität in drei Qualitätsklassen 32, 33, 34 umfassen, wobei die Klassifizierung eine zusätzliche Qualitätsklasse 33 mit einer mittleren Qualität vorsieht.

Zudem umfasst die ermittelte Bewertungskenngröße 29 auch die ermittelte Bewertungszahl. In einer alternativen Ausgestaltung kann die ermittelte Bewertungskenngröße 29 auch nur die Klassifizierung der Bewertungskenngröße 29 in die zwei oder drei Qualitätsklassen 32, 33, 34 umfassen.

Anschließend erfolgt in einem vierten Verfahrensschritt 103 ein Bereitstellen der zumindest eine Bewertungskenngröße 29. Das Bereitstellen erfolgt hierbei mittels der Bewertungseinheit 18 an die Benutzerschnittstelle 23, insbesondere an die Darstellungseinheit und/oder Ausgabeeinheit 24 der Benutzerschnittstelle 23.

In einem daran anschließenden fünften Verfahrensschritt 104 erfolgt eine Ausgabe der zumindest einen Bewertungskenngröße 29 an den Benutzer. Die Ausgabe erfolgt mittels der Darstellungseinheit und/oder Ausgabeeinheit 25 der Benutzerschnittstelle 23. Dabei werden visuelle Ausgabeelemente für die einzelnen Qualitätsklassen 32, 33, 34 der Bewertungskennzahl 29 generiert und ausgegeben. Das visuelle Ausgabeelement der Qualitätsklasse 32 mit der hohen Bildqualität kann dabei ein "gut" und/oder "+" und/oder einen lachenden Smiley und/oder einem grünen Punkt usw. umfassen. Das visuelle Ausgabeelement der Qualitätsklasse 34 mit der niedrigen und/oder unzureichenden Bildqualität kann dabei ein "schlecht" und/oder "-" und/oder einen weinenden Smiley und/oder einen roten Punkt usw. umfassen. Das visuelle Ausgabeelement der Qualitätsklasse 33 mit mittlerer Bildqualität kann dabei ein "mittel" und/oder einen neutral aussehenden Smiley und/oder einen orangen oder gelben Punkt usw. umfassen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einem Bereitstellen von zumindest einer Bewertungskenngröße einer Bildqualität von zumindest einem Magnetresonanzbild, umfassend die folgenden Schritte:
- Bereitstellen von zumindest einem Magnetresonanzbild für eine Bewertung der Bildqualität, wobei das Bereitstellen an eine Qualitätseinheit erfolgt,
- Bestimmen von zumindest zwei unterschiedlichen Qualitätsmaßen des zumindest einen Magnetresonanzbilds, wobei die Qualitätseinheit zwei oder mehr Qualitätsmodule umfasst und jedes der zwei oder mehr Qualitätsmodule zur Bestimmung eines der zwei oder mehr unterschiedlichen Qualitätsmaße ausgebildet ist, und Bereitstellen der zwei oder mehr Qualitätsmaße an eine Bewertungseinheit,
- Ermitteln der zumindest einer Bewertungskenngröße mittels der Bewertungseinheit, wobei die zumindest eine Bewertungskenngröße anhand der zwei oder mehr unterschiedlichen Qualitätsmaße ermittelt wird, und
- Bereitstellen der zumindest einen Bewertungskenngröße.

2. Verfahren nach Anspruch 1,
**dadurch gekennzeichnet, dass** das Bereitstellen der zumindest einer Bewertungskenngröße einer Bildqualität von zumindest einem Magnetresonanzbild während einer Magnetresonanzuntersuchung durchgeführt wird.

3. Verfahren nach einem der vorhergehenden Ansprüche, **dadurch gekennzeichnet, dass** zumindest eines der zwei oder mehr Qualitätsmodule ein trainiertes maschinelles Lernverfahren umfasst, wobei das trainierte maschinellen Lernverfahren dazu trainiert ist, ein Qualitätsmaß des zu bewertenden Magnetresonanzbilds durch Vergleich mit zumindest einem Referenzbild zu bestimmen.

4. Verfahren nach einem der vorhergehenden Ansprüche, **dadurch gekennzeichnet, dass** zumindest eines zwei oder mehr Qualitätsmodule ein trainiertes tiefes Lernverfahren umfasst, wobei das trainierte tiefe Lernverfahren dazu trainiert ist, ein Qualitätsmaß des zu bewertenden Magnetresonanzbilds ohne Vergleich mit einem Referenzbild zu bestimmen.

5. Verfahren nach einem der vorhergehenden Ansprüche, **dadurch gekennzeichnet, dass** die Bewertungseinheit ein maschinelles Lernverfahren zur Ermittlung der zumindest einen Bewertungskenngröße aus den zumindest zwei Qualitätsmaßen umfasst.

6. Verfahren nach einem der vorhergehenden Ansprüche, **dadurch gekennzeichnet, dass** die Bewertungseinheit zur Ermittlung der zumindest einen Bewertungskenngröße aus den zumindest zwei Qualitätsmaßen zumindest eine statistische Methode verwendet.

7. Verfahren nach einem der vorhergehenden Ansprüche, **dadurch gekennzeichnet, dass** zur Ermittlung der zumindest einen Bewertungskenngröße des zumindest einen Magnetresonanzbilds von der Bewertungseinheit weitere Bildparameter und/oder DICOM-Parameter berücksichtigt werden.

8. Verfahren nach einem der vorhergehenden Ansprüche, **dadurch gekennzeichnet, dass** in der Bewertungseinheit zur Ermittlung der zumindest einen Bewertungskenngröße eine Gewichtung der einzelnen ermittelten Qualitätsmaße erfolgt.

9. Verfahren nach einem der vorhergehenden Ansprüche, **dadurch gekennzeichnet, dass** in dem Bewertungseinheit zur Ermittlung der zumindest einen Bewertungskenngröße die zwei oder mehr Qualitätsmaße zur Klassifizierung der einzelnen Qualitätsmaße mit jeweils zumindest einem Eingangsschwellwert verglichen werden.

10. Verfahren nach einem der vorhergehenden Ansprüche, **dadurch gekennzeichnet, dass** die zumindest eine Bewertungskenngröße eine Klassifizierung der Bildqualität in zumindest zwei Qualitätsklassen umfasst, wobei eine Auswahl einer Qualitätsklasse in Abhängigkeit von einer ermittelten Bewertungszahl erfolgt.

11. Verfahren nach Anspruch 10,
**dadurch gekennzeichnet, dass** die zumindest eine Bewertungskenngröße die ermittelte Bewertungszahl umfasst.

12. Verfahren nach einem der Ansprüche 10 oder 11,
**dadurch gekennzeichnet, dass** die zumindest eine Bewertungskenngröße an einen Benutzer ausgegeben wird.

13. System mit einer Magnetresonanzvorrichtung, einer Qualitätseinheit und einer Bewertungseinheit, wobei das System zu einem Ausführen eines Verfahrens zu einem Bereitstellen von zumindest einer Bewertungskenngröße einer Bildqualität von zumindest einem Magnetresonanzbild nach einem der vorhergehenden Ansprüche ausgebildet ist.

14. Computerprogrammprodukt, welches ein Programm umfasst und direkt in einen Speicher einer programmierbaren Recheneinheit ladbar ist, mit Programmmitteln, um ein Verfahren zu einem Bereitstellen von zumindest einer Bewertungskenngröße einer Bildqualität von zumindest einem Magnetresonanzbild nach einem der Ansprüche 1 bis 12 auszuführen, wenn das Programm in der Recheneinheit ausgeführt wird.
